# EUROPEAN PATENT APPLICATION

(11) **EP 4 212 601 A1**
(43) Date of publication of application: **19.07.2023**
(21) Application number: 21866714.5
(22) Date of filing: 06.09.2021
(51) Int. Cl.: C09K 9/02, C09K 11/06, C07D 295/112, C09B 57/00, C09B 67/20, A61K 51/04, G01N 1/30, G01N 33/48, G01N 21/64

(54) **FLUORESCENT DYE AND METHOD FOR DETECTING TUMOR CELLS**

(30) Priority: 08.09.2020 JP 2020150559; 26.04.2021 JP 2021073923
(71) Applicant: National University Corporation Ehime University, Matsuyama-shi, Ehime 790-8577 (JP); National University Corporation Kochi University, Kochi-shi, Kochi 780-8520 (JP)
(72) Inventor: MURAKAMI,Masamoto, Toon-shi, Ehime 791-0295 (JP); KAWAKAMI,Ryosuke, Toon-shi, Ehime 791-0295 (JP); TSUDA,Teruko, Toon-shi, Ehime 791-0295 (JP); SAYAMA,Koji, Toon-shi, Ehime 791-0295 (JP); IMAMURA,Takeshi, Toon-shi, Ehime 791-0295 (JP); NIKO,Yosuke, Kochi-shi, Kochi 780-8520 (JP); INOUE,Kazuki, Kochi-shi, Kochi 780-8520 (JP); NAKAYAMA,Taku, Kochi-shi, Kochi 780-8520 (JP); HADANO,Shingo, Kochi-shi, Kochi 780-8520 (JP); WATANABE,Shigeru, Kochi-shi, Kochi 780-8520 (JP)
(74) Representative: Cabinet Beau de Loménie
(86) International application number: PCT/JP2021/032637
(87) International publication number: WO 2022/054755

(57) **Abstract**

The present invention relates to providing a novel method for easily detecting tumor cells in a tissue derived from an organism. The present invention includes a fluorescent dye for detecting tumor cells in a tissue, including a compound that exhibits solvatochromism. The present invention also includes a method for detecting tumor cells or specifying a tumor removal area, including applying the fluorescent dye to staining of a tissue derived from an organism and to an organism.

## Description

### TECHNICAL FIELD

The present invention relates to a fluorescent dye used for detecting tumor cells, and a method for detecting tumor cells.

### BACKGROUND ART

Various staining methods are used to detect tumor cells. For example, hematoxylin and eosin (HE) staining is the most widely used in pathological diagnosis and is a gold standard in many diagnostic methods. However, in HE staining, because in addition to tumor cells, normal tissues are also stained, and light permeability is deteriorated, slices of a tissue specimen are required. Because a thin sliced specimen has only two-dimensional information, production and observation of many sections are required to determine the distribution range of tumor cells. In addition, production of a tissue specimen requires a high degree of skill, is complicated, and takes a long time.

Further, in HE staining, the boundary between tumor cells and normal tissues may have a low contrast and be obscure.

A method of obtaining a high-contrast tumor cell image by staining a specific tissue with an antibody to which a fluorescent dye, a radionuclide, metal particles or the like is bound is also known. However, because the antibody part does not pass through the cell membrane, a treatment for increasing membrane permeability is required, and the operation is complicated. In addition, the reagent is expensive.

Meanwhile, there has also been reported a means for detecting a skin disease by irradiating light and utilizing a difference in Raman scattering between an abnormal tissue and a normal tissue without using a dye for staining (for example, Patent Document 1). However, in Raman scattering method, it is presumably difficult to determine the region of the tumor at the cellular level.

On the other hand, there are known some compounds that exhibit a phenomenon (solvatochromism) in which the absorption maximum wavelength, the fluorescence wavelength, or both of them are changed by the change in polarity of surrounding molecules such as a solvent. Compounds that exhibit solvatochromism are typically polarized by intramolecular charge transfer (ICT) upon photoexcitation. Because the stability of the excited state of the polarized molecules changes according to the degree of polarity of the solvent molecules, the energy difference between the excited state and the ground state of the molecules changes based on the polarity of the solvent.

As a compound that exhibits such solvatochromism, Non-Patent Document 1 describes 1-acetyl-6-piperidylpyrene (PK) and an aldehyde analog (PA) thereof. It is also described that normal tissues or HeLa cells were stained using PK.

### PRIOR ART DOCUMENTS

### PATENT DOCUMENT

Patent Document 1: JP-A-2018-201678

### NON-PATENT DOCUMENT

Non-Patent Document 1: Analytical Chemistry, 2020, Vol. 92, Issue 9, p. 6512-6520

### SUMMARY OF THE INVENTION

### PROBLEMS TO BE SOLVED BY THE INVENTION

However, Non-Patent Document 1 does not describe an example in which a tissue containing both normal cells and tumor cells was stained with a compound that exhibits solvatochromism. This Document does not describe that normal cells and tumor cells can be distinguished from each other by a difference in fluorescence wavelengths caused by solvatochromism.

In view of the above circumstances, an object of the present invention is to provide a novel method for easily detecting tumor cells in a tissue derived from an organism.

### MEANS FOR SOLVING THE PROBLEMS

The present inventors have found that tumor cells in a tissue can be easily detected by applying a fluorescent dye including a compound that exhibits solvatochromism to staining of a tissue derived from an organism, and have completed the present invention.

That is, the present invention provides the following fluorescent dye.
[1] A fluorescent dye for detecting tumor cells in a tissue derived from an organism, including a compound that exhibits solvatochromism.
[2] The fluorescent dye according to [1], wherein the compound includes a fused polycyclic π conjugated structure having 2 to 6 rings.
[3] The fluorescent dye according to [2], wherein the compound further includes at least one hydrophilic substituent containing at least one atom selected from the group consisting of boron, nitrogen, oxygen, phosphorus, sulfur, and halogen.
[4] The fluorescent dye according to any one of [1] to [3], wherein the compound is selected from a compound represented by Chemical Formula (II) below, 1-acetyl-6-piperidylpyrene, Nile Red, POLARIC (trademark), Laurdan, di-4-ANEPPDHQ, Prodan, and a derivative thereof. 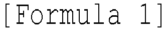 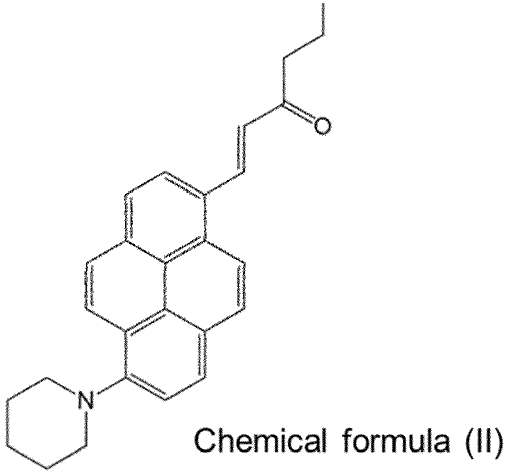
[5] The fluorescent dye according to any one of [1] to [4], wherein an absorption maximum wavelength of the compound is 300 to 600 nm in a 20 mM phosphate buffer (pH 7.4) at 25°C.
[6] The fluorescent dye according to any one of [1] to [5], wherein when a fluorescence maximum wavelength in dimethyl sulfoxide (DMSO) at 25°C is λ_{DMSO} and a fluorescence maximum wavelength in toluene at 25°C is λ_{Tol}, a difference between λ_{DMSO} and λ_{Tol} of the compound is 20 to 200 nm.
[7] A kit including the fluorescent dye according to any one of [1] to [6].

Further, the present invention provides the following method.
[8] A method for detecting tumor cells, including staining a tissue derived from an organism with a fluorescent dye including a compound that exhibits solvatochromism.
[9] A method for specifying a tumor removal area in a tissue in an organism, including staining the organism with a fluorescent dye including a compound that exhibits solvatochromism.
[10] Use of a compound that exhibits solvatochromism for detecting tumor cells in a tissue derived from an organism.
[11] Use of a compound that exhibits solvatochromism for specifying a tumor removal area.
[12] Use of a compound that exhibits solvatochromism for producing a composition for treatment or diagnosis of a tumor.
[13] A compound that exhibits solvatochromism for treatment or diagnosis of a tumor.

### EFFECT OF THE INVENTION

The tumor cells stained with the fluorescent dye of the present invention can be easily distinguished from normal cells and detected. The fluorescent dye of the present invention can easily and quickly stain a biological tissue.

### BRIEF DESCRIPTION OF THE DRAWINGS

Fig. 1 shows a chart of absorption spectrum measurement (left) and a chart of fluorescence spectrum measurement (normalized) (right) of PC (compound A) in an organic solvent in Examples.
Fig. 2A shows a two-photon micrograph of a Paget's disease lesion tissue in Test Example 1. The region corresponding to one tumor cell (Paget cell) is indicated by an arrowhead.
Fig. 2B shows a two-photon micrograph of a skin normal part tissue.
Fig. 3A shows a two-photon micrograph of a Paget's disease lesion tissue in Test Example 2. The arrowheads indicate Paget cells that proliferated in the epidermis, the arrowhead with an asterisk indicates a Paget cell that infiltrated and proliferated in the hair follicle, and the arrows indicate Paget cells that infiltrated and proliferated in the dermis.
Fig. 3B shows a two-photon micrograph of a Paget's disease lesion tissue in Test Example 2. The arrowheads indicate Paget cells infiltrating around a hair follicle.
Fig. 3C shows a two-photon micrograph of a melanoma lesion tissue in Test Example 2. The arrows indicate melanoma cells that proliferated in the epidermal basal cell layer, and the arrowheads indicate melanoma cells that proliferated in the dermis to form tumor honeycombs.
Fig. 4A shows two-photon micrographs of a skin normal part tissue in Test Example 3.
Fig. 4B shows two-photon micrographs of a Paget's disease lesion tissue in Test Example 3. The arrowheads indicate Paget cells that proliferated in the epidermis.
Fig. 4C shows two-photon micrographs of a melanoma lesion tissue in Test Example 3. The arrowheads indicate melanoma cells that proliferated in the epidermis.
Fig. 5 shows two-photon micrographs of a mucosal epithelial tissue of the rectum in Test Example 4. Panels A and B are normal part rectums, and panels C and D are well-differentiated adenocarcinoma parts of rectums.

### MODE FOR CARRYING OUT THE INVENTION

In the present specification, the "compound that exhibits solvatochromism" (in the present specification, abbreviated as "SC compound" or "solvatochromic compound") refers to a compound in which the absorption maximum wavelength or the fluorescence maximum wavelength, or both of them change due to the change in polarity (hydrophobicity) around the compound.

Without being limited to a specific mechanism of action, it is presumed that cell membrane environments (for example, polarity and orientation of molecules in the membrane, fluidity of the membrane and the like) are different between normal cells and tumor cells, thus the stabilities of the excited states of the SC compound of the present invention in the cell membrane become different as described above, and wavelengths of fluorescence to be emitted become different between tumor cells and normal cells.

### [Fluorescent dye]

The present invention includes a fluorescent dye including an SC compound, which is used for detecting tumor cells in a tissue derived from an organism..

### (Detection target)

The organism is not particularly limited as long as the organism is a multicellular animal, and is preferably a mammal (including human and non-human mammals), and more preferably a human.

The tissue is not particularly limited as long as the tissue has multiple cells. Examples of the tissue include skin, brain, spinal cord, esophagus, stomach, small intestine, large intestine, duodenum, rectum, liver, pancreas, gallbladder, bladder, kidney, heart, spleen, thymus, prostate, uterus, ovary, testis, breast, lungs, bronchus, eyeball, nose, sinus cavity, oral cavity, pharynx, salivary gland, thyroid gland, parathyroid gland, adrenal gland, muscle, bone marrow, blood vessel, nerve, lymph node, peritoneum, diaphragm, and blood. In one embodiment, the tissue is the skin, for example, epidermis or dermis, or a combination of both. In another embodiment, the tissue is the rectum.

The fluorescent dye of the present invention can be applied to, for example, a tissue separated from an organism by a surgical treatment such as extraction, excision, puncture, and blood collection, or a tissue obtained from feces, urine, sweat, other body fluids and the like.

In one embodiment, the form of the tissue can be appropriately selected according to the detection method, and can be, for example, an organ itself, or a thin sliced section or a three-dimensional specimen thereof.

Depending on the form, the tissue can be subjected to treatment such as fixation with formalin or the like, paraffin embedding, deparaffinization, dehydration, and clearing treatment.

The tumor detected by the fluorescent dye of the present invention can be benign or malignant (such as cells of cancer and sarcoma), but is preferably a malignant tumor. The type of tumor cells is also not particularly limited, and examples thereof include tumors generated in the above tissues.

In one embodiment, the tumor is a tumor of the skin. Examples of such tumor include sweat gland tumors (extramammary Paget's disease, mammary Paget's disease, eccrine porocarcinoma, microcystic adnexal carcinoma, mucinous carcinoma of skin and the like), malignant melanomas (melanomas), epidermal and hair follicle tumors (basal cell carcinoma, squamous cell carcinoma, actinic keratosis, Bowen's disease, leukoplakia, keratoacanthoma and the like), nervous system tumors (Merkel cell carcinoma, malignant peripheral nerve sheath tumor and the like), and mesenchymal tumors (dermatofibrosarcoma protuberans, isolated fibrotic tumor, muscle tumor, liposarcoma, angiosarcoma, Kaposi's sarcoma, spindle cell hemangioendothelioma, heterogeneous fibroxanthoma, epithelioid sarcoma, synovial sarcoma, undifferentiated pleomorphic cell sarcoma and the like). In a particular embodiment, the tumor is extramammary Paget's disease or malignant melanoma (melanoma).

### (Compound that exhibits solvatochromism (SC compound))

In the fluorescent dye of the present invention, the SC compound is not particularly limited as long as the SC compound is applicable to a tissue derived from an organism. Though one type of SC compounds can be used alone, or two or more types thereof can be used in combination, a clear stained image can be usually obtained by using one type of SC compounds alone.

As the size of the molecule of the SC compound is smaller, the SC compound is more likely to migrate to the cell membrane and the staining properties are improved. Thus, the size is preferably smaller. The molecular weight of the SC compound can be, for example, 800 or less, 700 or less, 600 or less, 500 or less, or 450 or less. The molecular weight of the SC compound can be, for example, 200 or more.

In one embodiment, the SC compound can be distributed in a cell membrane and inside a cell, and in a more particular embodiment, the SC compound can be inserted into a cell membrane.

In one embodiment, the SC compound exhibits strong fluorescence anisotropy in a cell membrane.

In one embodiment, the SC compound has a property of having a lower fluorescence intensity outside a cell as compared to a cell membrane and/or inside a cell. In a particular embodiment, substantially no fluorescence is observed outside a cell from the SC compound. Without particular limitation, for example, the fluorescence intensity of the SC compound outside a cell can be 50% or less, 40% or less, 30% or less, 20% or less, 10% or less, 5% or less, or 1% or less of the fluorescence intensity inside a cell. The comparison of the fluorescence intensities inside a cell and outside a cell is performed by irradiating a cell derived from a tissue of an object of the present invention with light having a wavelength capable of exciting the SC compound, acquiring a fluorescence microscope image under a condition capable of detecting light having a fluorescence maximum wavelength, and comparing average signal intensities of fluorescence inside the cell and fluorescence outside the cell.

In one embodiment, the SC compound includes a fused polycyclic π conjugated structure having 2, 3, 4, 5, or 6 rings. The fused polycyclic π conjugated structure is a polycyclic structure in which two or more rings are fused, including delocalized π electrons, and can contain one or more heteroatoms (nitrogen, oxygen, sulfur and the like), for example, like a benzophenoxazine ring. In a particular embodiment, the fused polycyclic π conjugated structure is a fused polycyclic aromatic hydrocarbon structure (for example, naphthalene, azulene, anthracene, phenanthrene, pyrene and the like). In a particular embodiment, the SC compound includes a pyrene skeleton (having 4 rings) as a fused polycyclic π conjugated structure. The SC compound containing a pyrene skeleton is particularly suitable for the use of the present invention because the fluorescence quantum yield is high even if the molecular size is reduced.

In one embodiment, the SC compound includes a structure in which 2, 3, 4, 5, or 6 conjugating rings are conjugated to each other. Examples of the conjugating rings include one or a combination of two or more selected from the group consisting of a benzene ring, a naphthalene ring, an anthracene ring, a phenanthrene ring, a fluorene ring, a pyridine ring, a thiophene ring, a pyrrole ring, a furan ring, a benzothiophene ring, a benzofuran ring, a benzopyrrole ring, an imidazole ring, a quinoline ring, an isoquinoline ring, a carbazole ring, a thiazole ring, and a dibenzothiophene ring.

The SC compound preferably further includes at least one hydrophilic substituent containing at least one atom selected from the group consisting of boron, nitrogen, oxygen, phosphorus, sulfur, and halogen from the viewpoint of improving water solubility and promoting dispersion in cell membranes and cells. Among them, from the viewpoint of suppressing the reaction with biomolecules, the hydrophilic substituent contained in the SC compound preferably contains one alone or two or more selected from the group consisting of a tertiary amino group, a quaternary ammonium group, and a carbonyl group (excluding an aldehyde group).

In a particular embodiment, from the viewpoint of staining properties, the SC compound is selected from PC that is a compound represented by Chemical Formula (II) below, 1-acetyl-6-piperidylpyrene (PK), Nile Red, POLARIC (trademark), Laurdan, di-4-ANEPPDHQ, Prodan, or a derivative thereof. In a further particular embodiment, the SC compound is selected from PC, PK, Nile Red, and POLARIC, is preferably PC or PK, and is more preferably PC.

1-Acetyl-6-piperidylpyrene (PK) is an SC compound represented by Chemical Formula (I) below. As shown in Table S1 of Analytical Chemistry, 2020, Vol. 92, Issue 9, p. 6512 -6520, PK exhibits a high fluorescence quantum yield in various solvents.

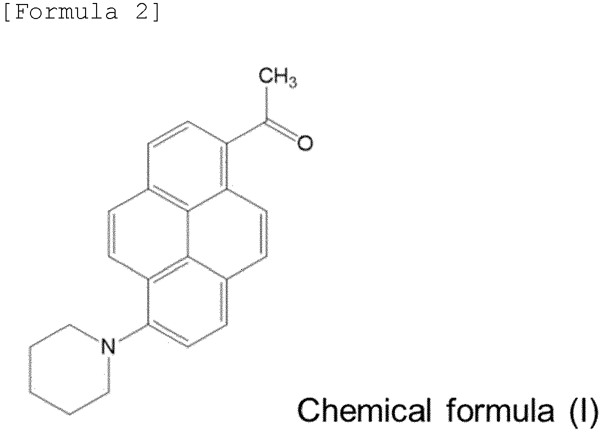

PC, also referred to as (E)-1-(6-(piperidin-1-yl)pyren-1-yl)hexa-1-en-3-one, is an SC compound represented by Chemical Formula (II) below. As shown in the Examples, PC exhibits a high fluorescence quantum yields in various solvents.

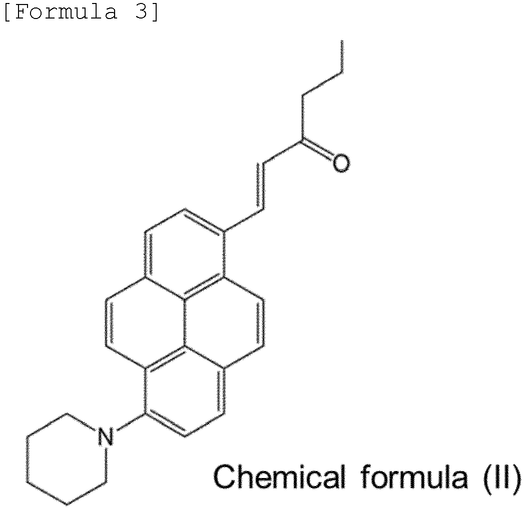

Nile Red is a compound with a CAS number of 7385-67-3. POLARIC is a compound described in Chem. Lett., 2011, Vol. 40, pp. 989-991 and a derivative thereof, and examples thereof include those sold as POLARIC series manufactured by Goryo Chemical, Inc. Laurdan is a compound with a CAS number of 74515-25-6. Details of the di-4-ANEPPDHQ are described, for example, in Biophys. J., 2006, Vol. 90, Issue 7, p. 2563-2575. Prodan is a compound with a CAS number of 70504-01-7.

Among the SC compounds, PC, PK, or Nile Red is preferred from the viewpoint of good photostability and high fluorescence quantum yield.

Further, PK and PC are more preferred because they are less adsorbed to non-specific tissues than Nile Red, the difference in the fluorescence wavelength between tumor tissues and normal tissues tends to be large, and background fluorescence can be suppressed.

In one embodiment, the absorption maximum wavelength of the SC compound is, for example, between 300 to 600 nm in a 20 mM phosphate buffer (pH 7.4) at 25°C. The absorption maximum wavelength of the SC compound can be, for example, within a range between any two numerical values selected from the group consisting of 300 nm, 310 nm, 320 nm, 330 nm, 340 nm, 350 nm, 360 nm, 370 nm, 380 nm, 390 nm, 400 nm, 410 nm, 420 nm, 430 nm, 440 nm, 450 nm, 460 nm, 470 nm, 480 nm, 490 nm, 500 nm, 510 nm, 520 nm, 530 nm, 540 nm, 550 nm, 560 nm, 570 nm, 580 nm, 590 nm, and 600 nm.

The absorption maximum wavelength of PK is 407 nm in a 20 mM phosphate buffer (pH 7.4) at room temperature as shown in Table S1 of Supporting Information in Analytical Chemistry, 2020, Vol. 92, Issue 9, p. 6512-6520.

In one embodiment, when the fluorescence maximum wavelength in methanol at 25°C is λ_{Met} and the fluorescence maximum wavelength in n-heptane at 25°C is λ_{Hep}, the difference between λ_{Met} and λ_{Hep} of the SC compound is, for example, 50 nm or more, 60 nm or more, 70 nm or more, 80 nm or more, 90 nm or more, 100 nm or more, or 110 nm or more. The difference is preferably larger because the larger the difference between λ_{Met} and λ_{Hep}, the more easily the tumor tissue is detected with high contrast with respect to the normal part tissue. The difference between λ_{Met} and λ_{Hep} of the SC compound can be 300 nm or less, 200 nm or less, or 150 nm or less.

In one embodiment, when the fluorescence maximum wavelength in dimethyl sulfoxide (DMSO) at 25°C is λ_{DMSO} and the fluorescence maximum wavelength in toluene at 25°C is λ_{Tol}, the difference between λ_{DMSO} and λ_{Tol} of the SC compound is, for example, 20 nm or more, 30 nm or more, 40 nm or more, 50 nm or more, 60 nm or more, 70 nm or more, 80 nm or more, 90 nm or more, or 100 nm or more. The difference is preferably larger because the larger the difference between λ_{DMSO} and λ_{Tol}, the more easily the tumor tissue is detected with high contrast with respect to the normal part tissue. The difference between λ_{DMSO} and λ_{Tol} of the SC compound can be 200 nm or less, 180 nm or less, 160 nm or less, 140 nm or less, or 120 nm or less.

The fluorescence maximum wavelengths of PK are λ_{Met} = 593 nm, λ_{Hep} = 474 nm, λ_{Tol} = 503 nm, and λ_{DMSO} = 558 nm as shown in Table S1 of Supporting Information in Analytical Chemistry, 2020, Vol. 92, Issue 9, p. 6512-6520.

In one embodiment, the two-photon absorption maximum wavelength of the SC compound is preferably, for example, between 600 to 1200 nm in a 20 mM phosphate buffer (pH 7.4) at 25°C. Such an SC compound is suitable for use in two-photon microscopy because the SC compound can be excited by light of a wavelength that is poorly absorbed by biological substances in tissues.

### (Other compositions)

In one embodiment, the fluorescent dye consists of the SC compound alone.

In one embodiment, the fluorescent dye can further include any one alone or a combination of two or more of a pH buffer, a surfactant, a salt, a solvent, a dye composition different from the SC compound and the like.

Examples of the pH buffer include one alone or a combination of two or more selected from the group consisting of tris(hydroxymethyl)aminomethane; Good's buffers (HEPES, MOPS and the like); and a pH buffer containing citric acid, acetic acid, lactic acid, oxalic acid, phthalic acid, imidazole, triethanolamine, diethanolamine, glycine, boric acid, phosphoric acid, or carbonic acid.

Examples of the solvent include one alone or a combination of two or more selected from the group consisting of water, ethanol, methanol, 2-propanol, dimethyl sulfoxide (DMSO), N,N-dimethylformamide, acetonitrile, acetone, ethyl acetate, tetrahydrofuran, and 1,2-dichloroethane.

The dye composition different from the SC compound is not particularly limited as long as the dye composition does not interfere with the detection of tumor cells by the SC compound. For example, nuclear staining dyes such as propidium iodide (PI), ethidium bromide, acridine orange, DAPI, and Hoechst hardly disturb staining of SC compounds, and can be suitably used. As the dye composition different from the SC compound, for example, dye compositions used for various tissue stainings such as hematoxylin/eosin (HE) staining, azan staining, Masson's trichrome staining, elastica/Wangeson staining, silver plating staining, Victoria blue staining, PAM staining, PTAH staining, Sudan III staining, oil red O staining, PAS staining, alcian blue staining, toluidine blue staining, colloidal iron staining, mucicarmine staining, Congo red staining, Dylon staining, Grimelius staining, Fontana Masson staining, Kossa staining, Berlin blue staining, Bodian staining, Kluber/Valera staining, and Giemsa staining can be used. These different dye compositions can be used singly or in combination of two or more types thereof.

### (Dosage form)

The dosage form of the fluorescent dye of the present invention is not particularly limited, and examples thereof include a solid such as a powder and a liquid.

### (Use)

When a tissue is stained with the fluorescent dye of the present invention, the SC compound can be distributed in both tumor cells and cells of normal tissues, and the fluorescence wavelength of the SC compound in the tumor cells shifts from the fluorescence wavelength in cells of normal tissues. Thus, by appropriately selecting the wavelength of fluorescence to be detected, tumor cells are detected with high contrast relative to normal tissues. The fluorescent dye of the present invention can be used for inspection or diagnosis of a tumor, particularly a malignant tumor.

The fluorescent dye of the present invention can be applied to an organism itself or a part of an organism that is not separated from the organism.

When the fluorescent dye of the present invention is applied to an organism, the fluorescent dye can be used for diagnosis of a tumor, particularly a malignant tumor. Further, to specify the removal area of a tumor, particularly a cancer, of an organism or to confirm whether a tumor remains in treatment of a tumor, the fluorescent dye of the present invention can be applied to an organism before, at the time of, or after the removal treatment as a part of treatment.

The fluorescent dye of the present invention can be, for example, a pharmaceutical agent or a quasi-drug, in addition to a reagent used for clinical examination, basic research and the like.

### (Kit)

The fluorescent dye of the present invention can also be combined with, for example, a reagent or an instrument for staining or preparing a tissue specimen to form a kit. In a particular embodiment, the kit includes a reagent for preparing a stain solution. In a further particular embodiment, the reagent for preparing a stein solution can contain, for example, one alone or a mixture of two or more selected from the group consisting of the pH buffers, a surfactant, a salt, a solvent, and another dye composition, described above.

### [Method for detecting tumor cells in tissue]

The present invention includes a method for detecting tumor cells, including the step of: staining a tissue derived from an organism with a fluorescent dye including a compound that exhibits solvatochromism.

Examples of the fluorescent dye including a compound that exhibits solvatochromism include those described in the above [Fluorescent dye].

Examples of the tissue derived from an organism include those described in the section of [Fluorescent dye] above. The method of the invention can include the step of preparing such tissues.

In one embodiment, the method of the present invention is applied to a tissue such as an internal or other organ itself or a three-dimensional fragment thereof. In this embodiment, a step of clearing the tissue before staining is preferably included. Examples of the tissue clearing method include TDE method, LUCID method, CLARITY method, PACT/PARS method, CUBIC method, 3DISCO method, Scale method, ScaleS method, SeeDB method, FocusClear method, Clear method, BABB method, iDISCO method, and uDISCO method. These clearing methods are described, for example, in Cell Chem. Biol. 2016, Vol. 23, 137-157, Laser Photonics Rev. 2019, Vol. 13, 1800292.

In one embodiment, the method of the present invention is applied to an organism itself or a part of an organism that is not separated from the organism.

As one embodiment, the method of the present invention is applied to a thin sliced section. The thin sliced section can optionally be subjected to treatments such as fixation, dehydration, dealcoholization, paraffin penetration, paraffin embedding, deparaffinization, immersion, and staining using the various tissue staining methods described above that are usually used in clinical examinations.

Staining is usually performed by bringing a stain including an SC compound into contact with a tissue. The concentration of the SC compound in the stain is adjusted to, for example, 0.001 mg/mL or more, 0.01 mg/mL or more, 0.1 mg/mL or more, 0.2 mg/mL or more, 0.3 mg/mL or more, 0.4 mg/mL or more, 0.5 mg/mL or more, 0.6 mg/mL or more, 0.7 mg/mL or more, 0.8 mg/mL or more, 0.9 mg/mL or more, or 1 mg/mL or more with respect to the total amount of the stain. The concentration of the SC compound in the stain is adjusted to, for example, 500 mg/mL or less, 200 mg/mL or less, 100 mg/mL or less, 50 mg/mL or less, 20 mg/mL or less, 10 mg/mL or less, 5 mg/mL or less, or 2 mg/mL or less with respect to the total amount of the stain.

The temperature at the time of staining is not particularly limited, and is, for example, 0 to 80°C, 4 to 50°C, or 20 to 45°C, and preferably 35°C to 42°C.

The time for bringing a stain into contact with a tissue is, for example, 1 minute or more, 10 minutes or more, 20 minutes or more, 1 hour or more, 2 hours or more, 1 day or more, or 2 days or more at 20 to 40°C, and is, for example, 14 days or less or 7 days or less.

In one embodiment, the time for bringing a stain into contact with a tissue is, for example, 12 hours or less or 6 hours or less, preferably 2 hours or less, more preferably 1 hour or less, still more preferably 30 minutes or less, and still more preferably 10 minutes or less at 35 to 40°C, and can be, for example, 1 minute or more, 2 minutes or more, 5 minutes or more, or 10 minutes or more.

The tissue stained with a stain including an SC compound can be used as it is for detecting tumor cells, but can optionally be subjected to a treatment such as staining with another dye composition before detection.

The method of the present invention can further include the step of detecting tumor cells. The detection of tumor cells is performed, for example, by exciting the SC compound with light of an appropriate wavelength and detecting emitted fluorescence. For the detection, for example, a confocal laser scanning microscope can be used, and a microscope capable of multiphoton excitation such as a two-photon microscope is used depending on the thickness of the section. For example, when a compound of Chemical Formula (II) (PC) or 1-acetyl-6-piperidylpyrene (PK) is used as the SC compound, measurement by a two-photon microscope is suitable as shown in Examples. Among them, PC is particularly suitable for detecting a tumor in a deeper portion because the fluorescence maximum wavelength is larger than PK.

In one embodiment, tumor cells are detected by selecting fluorescence including one specific wavelength such that a contrast is obtained between tumor cells and cells of a normal tissue and measuring the fluorescence intensity thereof.

In another embodiment, the detection of tumor cells is performed by multi-wavelength measurement. That is, tumor cells are detected by detecting fluorescence including two or more different specific wavelengths and integrating the respective fluorescence intensities.

As one embodiment, when PC or PK is used as the SC compound, the fluorescence to be detected includes one or two or more wavelengths selected from, for example, a range of 450 to 550 nm, a range of 480 nm to 520 nm, or a range of 490 nm to 500 nm. In a particular embodiment, the fluorescence to be detected includes light of 495 nm.

According to the method of detection of the present invention, as described in the section of [Fluorescent dye] above, inspection of a tumor, specification of a tumor removal area, diagnosis of a tumor, or treatment of a tumor can be performed.

### [Preferred embodiment of the invention]

The present invention includes the following preferred embodiments.
<1> A fluorescent dye for detecting tumor cells in a tissue derived from an organism, including a compound that exhibits solvatochromism.
<2> A kit including the fluorescent dye according to <1>.
<3> A method for detecting tumor cells, including staining a tissue derived from an organism with a fluorescent dye including a compound that exhibits solvatochromism.
<4> A method for specifying a tumor removal area in a tissue in an organism, including staining the organism with a fluorescent dye including a compound that exhibits solvatochromism.
<5> A method for treating or diagnosing a tumor, including: staining a tissue derived from an organism or an organism with a fluorescent dye including a compound that exhibits solvatochromism; and detecting a tumor based on a stained image.
<6> Use of a compound that exhibits solvatochromism for detecting tumor cells in a tissue derived from an organism.
<7> Use of a compound that exhibits solvatochromism for specifying a tumor removal area.
<8> Use of a compound that exhibits solvatochromism for producing a composition for treatment or diagnosis of a tumor.
<9> A compound that exhibits solvatochromism for treatment or diagnosis of a tumor.
<10> In <1> to <9> above, the compound includes a fused polycyclic π conjugated structure having 2 to 6 rings, and preferably includes a pyrene skeleton.
<11> In <1> to <10> above, the compound further includes at least one hydrophilic substituent containing at least one atom selected from the group consisting of boron, nitrogen, oxygen, phosphorus, sulfur, and halogen.
<12> In <1> to <11> above, a molecular weight of the compound is 800 or less, 700 or less, 600 or less, 500 or less, or 450 or less, and 200 or more.
<13> In <1> to <12> above, the compound is selected from a compound represented by Chemical Formula (II) below, 1-acetyl-6-piperidylpyrene, Nile Red, POLARIC (trademark), Laurdan, di-4-ANEPPDHQ, Prodan, and a derivative thereof, preferably selected from a compound represented by Chemical Formula (II), 1-acetyl-6-piperidylpyrene, Nile Red, and POLARIC, more preferably a compound represented by Chemical Formula (II) or 1-acetyl-6-piperidylpyrene, and still more preferably a compound represented by Chemical Formula (II). 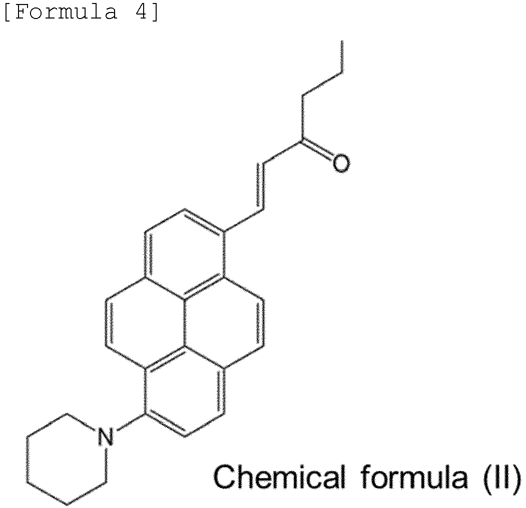
<14> In <1> to <13> above, an absorption maximum wavelength of the compound is 300 to 600 nm in a 20 mM phosphate buffer (pH 7.4) at 25°C, and is within a range between any two numerical values selected from the group consisting of, for example, 300 nm, 310 nm, 320 nm, 330 nm, 340 nm, 350 nm, 360 nm, 370 nm, 380 nm, 390 nm, 400 nm, 410 nm, 420 nm, 430 nm, 440 nm, 450 nm, 460 nm, 470 nm, 480 nm, 490 nm, 500 nm, 510 nm, 520 nm, 530 nm, 540 nm, 550 nm, 560 nm, 570 nm, 580 nm, 590 nm, and 600 nm.
<15> In <1> to <14>, when the fluorescence maximum wavelength in methanol at 25°C is λ_{Met} and the fluorescence maximum wavelength in n-heptane at 25°C is λ_{Hep}, the difference between λ_{Met} and λ_{Hep} of the SC compound is 50 nm or more, 60 nm or more, 70 nm or more, 80 nm or more, 90 nm or more, 100 nm or more, or 110 nm or more, and 300 nm or less, 200 nm or less, or 150 nm or less.
<16> In <1> to <15> above, when the fluorescence maximum wavelength in dimethyl sulfoxide (DMSO) at 25°C is λ_{DMSO} and the fluorescence maximum wavelength in toluene at 25°C is λ_{Tol}, the difference between λ_{DMSO} and λ_{Tol} of the SC compound is 20 nm or more, 30 nm or more, 40 nm or more, 50 nm or more, 60 nm or more, 70 nm or more, 80 nm or more, 90 nm or more, or 100 nm or more, and 200 nm or less, 180 nm or less, 160 nm or less, 140 nm or less, or 120 nm or less.
<17> In <1> to <16> above, the organism is a human or a non-human mammal.
<18> In <1> to <17> above, a tissue in which the tumor or tumor cells are present is selected from skin, brain, spinal cord, esophagus, stomach, small intestine, large intestine, duodenum, rectum, liver, pancreas, gallbladder, bladder, kidney, heart, spleen, thymus, prostate, uterus, ovary, testis, breast, lungs, bronchus, eyeball, nose, sinus cavity, oral cavity, pharynx, salivary gland, thyroid gland, parathyroid gland, adrenal gland, muscle, bone marrow, blood vessel, nerve, lymph node, peritoneum, diaphragm, and blood.
<19> In <1> to <18> above, the tumor is a malignant tumor.
<20> In <1> to <19> above, the tissue in which the tumor or tumor cells are present is skin, and the tumor is selected from a sweat gland tumor, a malignant melanoma, an epidermal and hair follicle tumor, a nervous system tumor, and a mesenchymal tumor, and is preferably extramammary Paget's disease or malignant melanoma.

### EXAMPLES

### [1. Material]

### [1-1. Synthesis and analysis of PC]

The synthesis of PC was performed as in the following scheme.

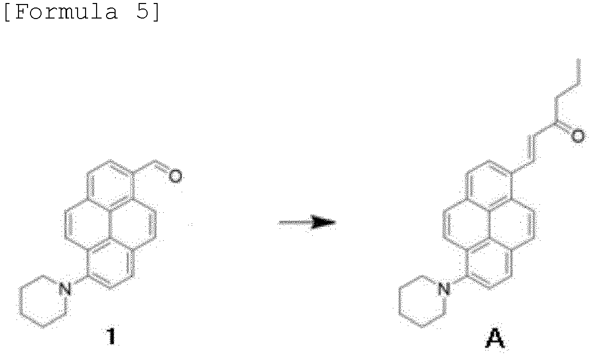

A 1 M aqueous sodium hydroxide solution (1 mL) and dehydrated ethanol (6 mL) were added to compound 1 which is a known pyrene derivative (100 mg, 0.32 mmol) and 2-pentanone (47 µL, 0.48 mmol), and the obtained solution was heated and stirred at 60°C for 4 hours under an argon atmosphere.

Then, water was added to the solution, and the resulting precipitate was collected by filtration.

The obtained precipitate was purified by silica gel column chromatography (dichloromethane : hexane = 2 : 1), and further recrystallized with acetonitrile to obtain a target compound A (PC). (yield amount: 20 mg, yield percent: 160)

### (¹H-NMR analysis and ¹³C-NMR analysis)

¹H-NMR analysis and ¹³C-NMR analysis were performed using a nuclear magnetic resonance apparatus (JMN-LA 500 manufactured by JEOL Ltd.). The results obtained in the compound A are shown below.
· ¹H NMR (500 MHz, CDCl₃): δ (ppm) = 8.71 (d, J = 15.6 Hz, 1H), 8.46 (d, J = 9.2 Hz, 1H), 8.33 (d, J = 9.2 Hz, 1H), 8.25 (d, J = 9.2 Hz, 1H), 8.15 (d, J = 8.1 Hz, 1H), 8.09 (d, J = 8.1 Hz, 1H), 8.08 (d, J = 9.2 Hz, 1H), 8.03 (d, J = 9.2 Hz, 1H), 7.74 (d, J = 8.1 Hz, 1H), 7.01 (d, J = 15.6 Hz, 1H), 3.22 (s, 4H), 2.78 (t, J = 7.4 Hz, 2H), 1.93-1.95 (m, 4H), 1.77-1.85 (m, 2H), 1.73 (s, 2H), 1.06 (t, J = 7.33 Hz, 1H).
· ¹³C NMR (CDCl₃, TMS) δ (ppm) = 14.11, 18.12, 24.67, 26.84, 43.49, 55.21, 117.53, 120.49, 124.26, 124.48, 124.71, 125.09, 125.68, 126.02, 126.31, 126.51, 126.57, 127.66, 128.75, 130.80, 133.21, 139.25, 150.33, 200.49.

### (High-resolution mass spectrometry)

The high-resolution mass spectrometry was performed using a high-resolution mass spectrometer (JMS-700 manufactured by JEOL Ltd.). The results obtained in the compound A are shown below.
· HRMS (ESI⁺), calcd for C₂₅H₂₃NO [M+Na]⁺ 404.1985, found 404.1981.

### (Measurement of photophysical properties of PC in organic solvent)

The absorption spectrum and fluorescence spectrum of the PC in organic solvents were measured.

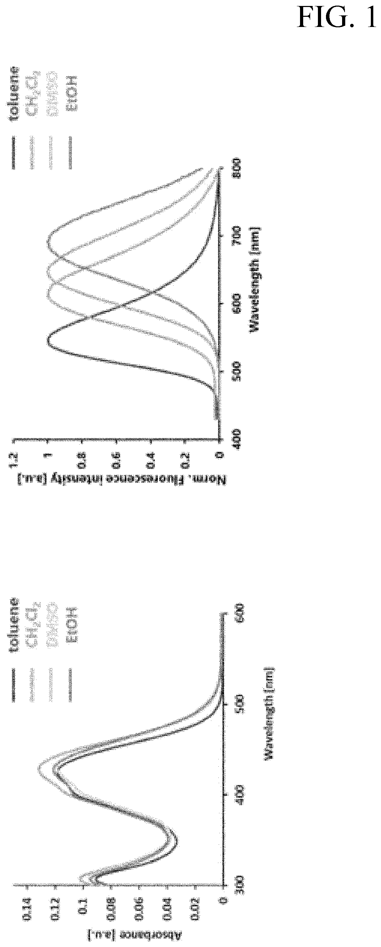

The absorption spectrum and the fluorescence spectrum were measured using a UV-Visible/NIR spectrophotometer (V-670 manufactured by JASCO Corporation) and a spectrofluorometer (FP 6600 manufactured by JASCO Corporation), respectively. The fluorescence quantum yield was measured using an absolute PL quantum yield spectrometer (Hamamatsu Photonics K.K.: C 9920-02V). The concentration of the compound A was 5 µM for each solvent. For solvent species, toluene, dichloromethane (DCM), DMSO, and ethanol were used. The obtained results are shown in Fig. 1 and Table 1. The absorption maximum wavelength and the fluorescence maximum wavelength of the compound A in a 20 mM phosphate buffer (pH 7.4) at 25°C were 421 nm and 621 nm, respectively.

**[Table 1] Table. 1 Maximum absorption wavelength, maximum fluorescence wavelength, and fluorescence quantum yield of compound A in organic solvent**

| Solvent | Maximum absorption wavelength (nm) | Maximum fluorescence wavelength (nm) | Fluorescence quantum yield (%) |
|---|---|---|---|
| Toluene | 424 | 546 | 82 |
| DCM | 427 | 615 | 86 |
| DMSO | 429 | 647 | 79 |
| EtOH | 427 | 691 | 20 |

As shown in Fig. 1 and Table 1, the absorption maximum wavelengths were almost the same value irrespective of the solvent species, but the fluorescence maximum wavelengths were different depending on the solvent species. More specifically, in polar solvents, an increase in the fluorescence wavelength was observed, and fluorescence solvatochromism sensitive to solvent polarity was observed in polar solvents.

### [1-2. Preparation of SC compounds other than PC]

As PK, one synthesized by the method described in Analytical Chemistry, 2020, Vol. 92, Issue 9, p. 6512-6520 was used. As Nile Red, one manufactured by Tokyo Chemical Industry Co., Ltd. was used. As POLARIC, POLARIC (trademark) 500 BCS (manufactured by Goryo Chemical, Inc.) was used.

### [1-3. Preparation of staining solutions]

The staining solution of Test Examples 1 and 2 was prepared by dissolving PK in DMSO at 1 mg/mL. Each of staining solutions of Test Example 3 and Test Example 4 was a DMSO solution containing 1 mM of an SC compound, and was diluted 100 times for use of staining.

### [1-4. Tissue used]

As the skin normal part tissue, the extramammary Paget's disease lesion tissue, and the melanoma lesion tissue used in Test Examples, whole tissue sections of the tissues obtained by excision were used. A dermatologist has confirmed in advance the identification of each tissue. Also, a normal part tissue and a tumor tissue of the rectum were also prepared by whole-sectioning of the tissues obtained by excision, and a specialist has confirmed the identification of each tissue. The collection and use of the tumor tissues were approved by the Clinical Research Ethics Review Board at Ehime University Hospital (No. 1802009) and were conducted according to its study protocol.

### [2. Test Example 1: Observation of tumor cells by PK staining of thin sliced section]

The extramammary Paget's disease lesion tissue and the normal part tissue were embedded in paraffin and then cut out to prepare tissue sections. The preparation of the sections (thickness: 5 um) was performed by the procedure usually performed in histopathological examination. For staining with the PK staining solution, deparaffinization was performed by a conventional method, and then staining was performed by immersion in the PK staining solution at room temperature for several days to one week. In practice, the tissues were sufficiently stained by immersion in the PK staining solution at room temperature for 2 to 3 days.

For microscopy, a two-photon microscope A1R MP + (NIKON) was used. In the measurement, a 960 nm laser light source and a 495 nm filter were used for PK, and a 561 nm filter was used for PI. Scanning was performed to a depth of 100 um of the sample. The three-dimensional reconstructed image is obtained by processing the obtained data with the software included.

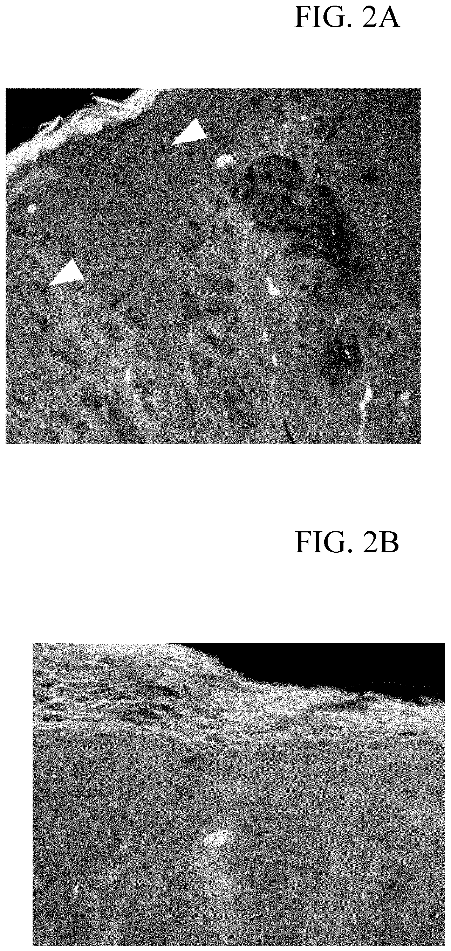

A part of the images of 10 um thickness from the three-dimensional reconstructed image of eachtissue are shown in Figs. 2A and 2B. As shown in Fig. 2A, the fluorescence wavelength shifted and the signal level decreased in the parts where the tumor cells (Paget cells) exist. As a result, the cells on the back side of the tumor cells were seen through, and there appeared to be an apparent void. On the other hand, in the surrounding normal cells, the contour of the cell membrane was stained with PK. On the other hand, as shown in Fig. 2B, in the normal part tissue, there was no such a region where the signal level was low.

From the above, it became clear that tumor cells can be distinguished from normal cells by staining cells with PK and appropriately selecting the excitation wavelength and the wavelength of fluorescence to be detected. Further, PK was shown to be inserted into cell membranes.

### [3. Test Example 2: Observation of tumor cells by PK staining of cleared three-dimensional tissue]

Derived extramammary Paget's disease lesion tissue having a thickness of 500 um and malignant melanoma lesion tissue having a thickness of 100 um were subjected to a clearing treatment without being subjected to fixation and thinly slicing, and then stained with PK and propidium iodide (PI). Clearing treatment was performed by using LUCID method, according to the procedure described in Sawada K, Kawakami R, Shigemoto R, and Nemoto T. Eur. J. Neurosci., 2018, Vol. 47, No. 9, 1033-1042. The cleared tissue was stained with a PK staining as in Test Example 1. PI staining was performed by a method usually performed in tissue staining. As conditions for microscopy, the same conditions as in Test Example 1 were used for detection of PK signals.

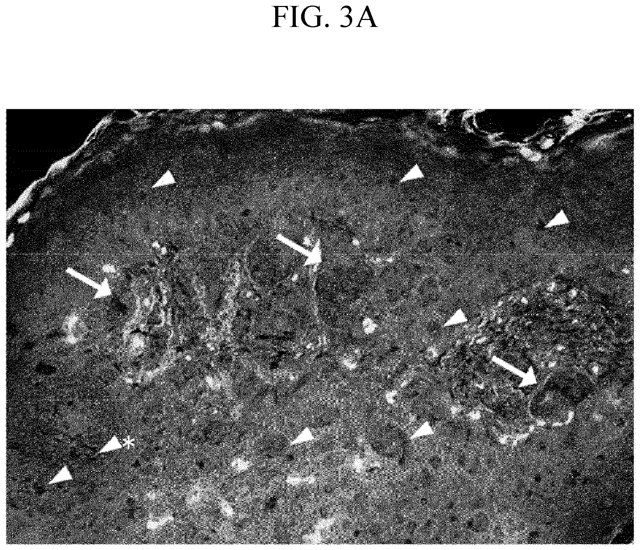

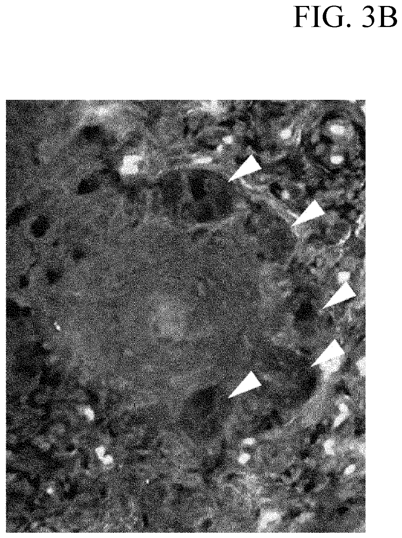

Images of fragments having a thickness of 10 um of three-dimensional reconstructed images of the extramammary Paget's disease lesion tissue are shown in Figs. 3A and 3B. By using a two-photon microscope, three-dimensional PK stained images were successfully obtained without thinly slicing even for tissues as thick as 500 µm. Tumor cells (Paget cells) were successfully detected even when they were scattered one by one. In a region corresponding to tumor cells having a low level of fluorescence signal of 495 nm, only nuclei were observed to be PI-stained. Thus, it was confirmed that cells were actually present in the region.

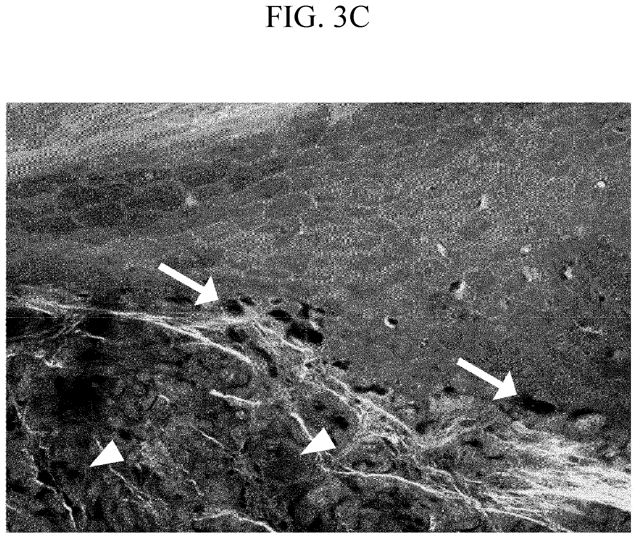

A three-dimensional reconstructed image of the malignant melanoma lesion tissue is shown in Fig. 3C. Same as the Paget's disease lesion, fluorescence signal caused by PK staining was also not observed in malignant melanoma cells, and only nuclei were observed by PI staining. As described above, even when the types of tumor cells are different, the fluorescent dye of the present invention can specifically detect tumor cells.

These observed tissues included normal cells of the epidermis, dermis, and hair follicle, and signals caused by PK staining were significantly observed in all of these cells. Thus, it is understood that PK staining can distinguish and detect tumor cells from these various normal cells.

### [4. Test Example 3: Comparison of stainings between normal tissue and tumor tissue with various SC compounds]

Normal part tissue of the skin and extramammary Paget's disease lesion tissue cleared by LUCID method in the same manner as in Test Example 2 were stained with a staining solution containing PK, PC, Nile Red, or POLARIC. Each stained tissue section was observed according to the method of two-photon microscopy of Test Example 1. The images were produced by coloring a fluorescent wavelength of 492 nm or less in cyan, 500 to 550 nm in green, 560 to 593 nm in yellow, and 593 nm or more in red. Brightness and contrast of the illustrated images were appropriately corrected.

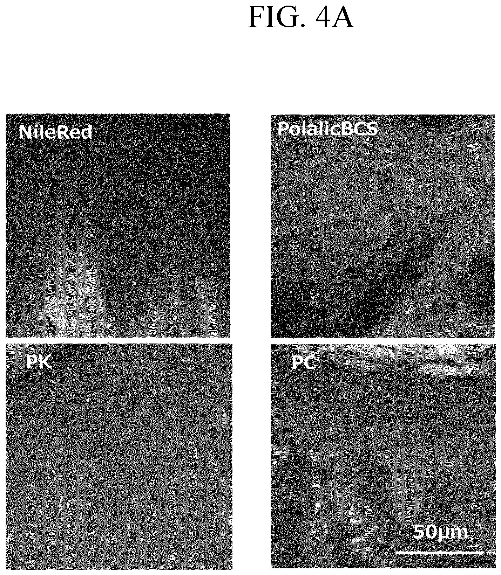

In the normal part tissue, as shown in Fig. 4A, a planar structure filled with cells without gaps was obtained as an observation image regardless of which staining solution was used.

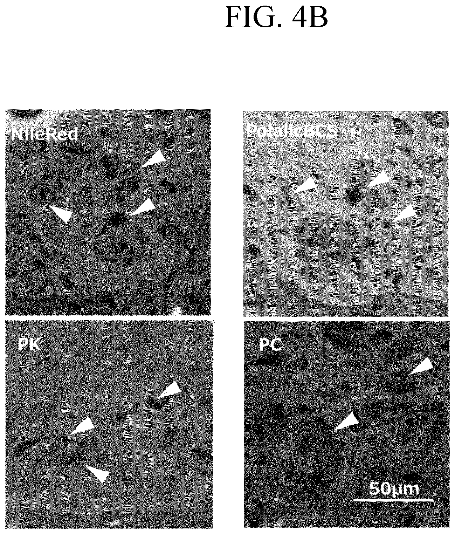

On the other hand, in the extramammary Paget's disease lesion tissue, as shown in Fig. 4B, as a result of shifting of the fluorescence wavelength of the region corresponding to tumor cells, void portions similar to those in Test Example 1 was observed. In comparison of the imaging performances for fluorescence observation, PC had the best performance, followed by PK, Nile Red, and POLARIC in this order. In that, the higher the fluorescence intensity and the contrast of staining are, the higher the imaging performance was judged. In Nile Red and POLARIC, the fluorescence intensity of the raw image was lower than those of PK and PC, and brightness and contrast adjustment was required for tumor identification.

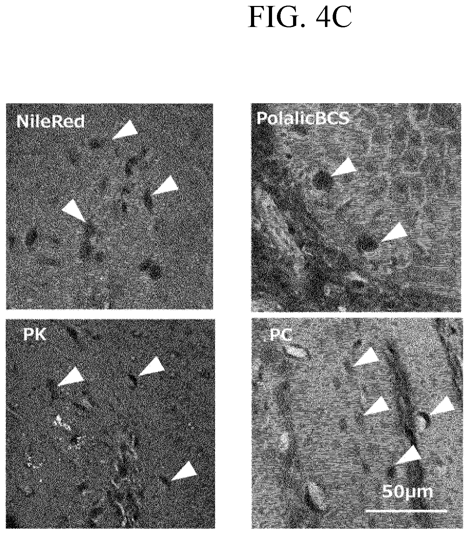

Melanoma lesion tissue of the skin cleared by the same method were similarly stained and observed with a two-photon microscope. As a result, as shown in Fig. 4C, the tumor cell portions were observed in void shapes as in the Paget's disease lesion.

In comparison of the imaging performances for fluorescence observation, PC had the best performance, followed by PK, POLARIC, and Nile Red in this order. In Nile Red and POLARIC, the shift of the fluorescence wavelength due to solvatochromism was relatively small, and thus it was found that the degree of the shift of the fluorescence wavelength was related to the imaging performance.

It was also found that when POLARIC was adsorbed to a normal tissue, a strong tendency was emitted in the observed wavelength region. In POLARIC, the background tended to appear when a tumor tissue was stained, and in order to obtain a good image of a lesion, it was necessary to lower the fluorescence intensity of the entire image, for example, by lowering the laser power to 1/4 (2.5%) of PK and PC. On the other hand, in other SC compounds, an image with a high imaging performance was obtained without particularly correcting the background intensity.

### [5. Test Example 4: Comparison of stainings between normal tissue and tumor tissue in rectum]

The normal part tissue and the well-differentiated adenocarcinoma lesion tissue of the rectum cleared by LUCID method in the same manner as in Test Example 2 were stained with PK and Hoechst. Each stained tissue section was observed according to the method of two-photon microscopy of Test Example 1.

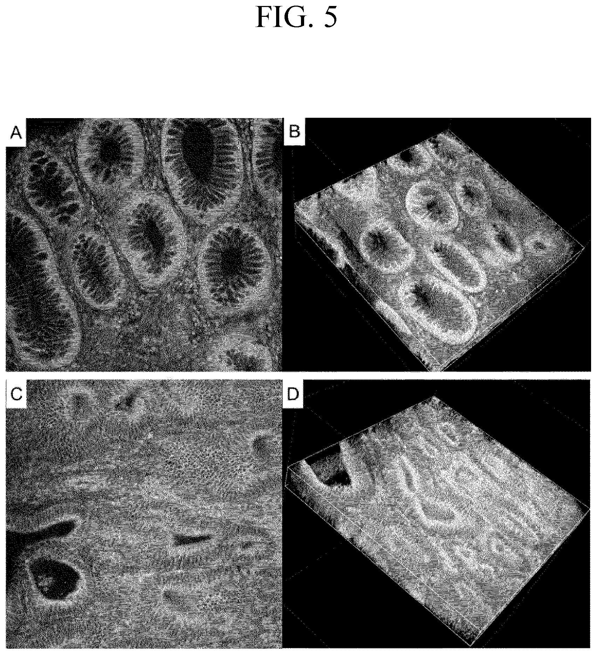

The results are shown in Fig. 5. In normal rectal mucosal epithelium (columnar epithelium), cell membranes were positive for PK staining, cytoplasm was poorly stained, and only nuclei stained with Hoechst were observed. On the other hand, in the tumor lesion, the cytoplasmic staining property by PK staining was high, and the distribution of cells was observed. That is, contrary to the cases of Test Examples 1 to 3, fluorescence of a rectal tumor tissue was observed, so that a stained image in which a tumor tissue is clearly distinguishable from a normal tissue was obtained.

## Claims

1. A fluorescent dye for detecting tumor cells in a tissue derived from an organism, comprising a compound that exhibits solvatochromism.

2. The fluorescent dye according to claim 1, wherein the compound includes a fused polycyclic π conjugated structure having 2 to 6 rings.

3. The fluorescent dye according to claim 2, wherein the compound further includes at least one hydrophilic substituent containing at least one atom selected from the group consisting of boron, nitrogen, oxygen, phosphorus, sulfur, and halogen.

4. The fluorescent dye according to any one of claims 1 to 3, wherein the compound is selected from a compound represented by Chemical Formula (II) below, 1-acetyl-6-piperidylpyrene, Nile Red, POLARIC (trademark), Laurdan, di-4-ANEPPDHQ, Prodan, and a derivative thereof.

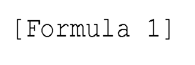

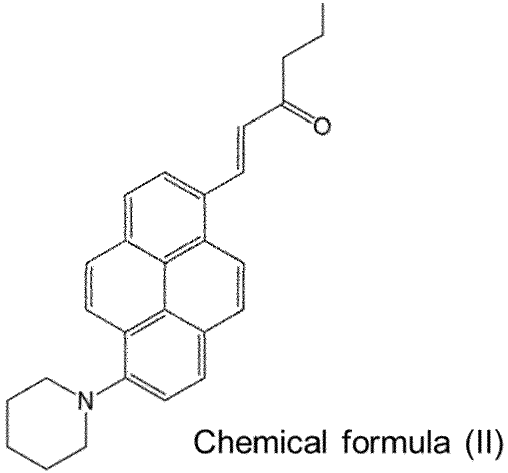

5. The fluorescent dye according to any one of claims 1 to 4, wherein an absorption maximum wavelength of the compound is 300 to 600 nm in a 20 mM phosphate buffer (pH 7.4) at 25°C.

6. A kit comprising the fluorescent dye according to any one of claims 1 to 5.

7. A method for detecting tumor cells, comprising staining a tissue derived from an organism with a fluorescent dye including a compound that exhibits solvatochromism.
